# EUROPEAN PATENT SPECIFICATION

(11) **EP 1 945 298 B1**
(45) Date of publication and mention of the grant of the patent: **17.04.2013**
(21) Application number: 06812517.8
(22) Date of filing: 09.11.2006
(51) Int. Cl.: A61B 18/14, A61N 1/06

(54) **RADIO FREQUENCY ABLATION ELECTRODE FOR SELECTED TISSUE REMOVAL**
HOCHFREQUENZ-ABLATIONSELEKTRODE ZUR SELEKTIVEN GEWEBEENTFERNUNG
ÉLECTRODE D'ABLATION À FRÉQUENCE RADIO POUR ENLÈVEMENT DE TISSU SÉLECTIONNÉ

(30) Priority: 09.11.2005 KR 20050106763
(43) Date of publication of application: 23.07.2008
(73) Proprietor: Korea University Industrial & Academic Collaboration Foundation, Seoul 136-701 (KR)
(72) Inventor: LEE, Sang-Heon, Gangnam-gu, Seoul 135-272 (KR); SUN, Kyung, Nowon-gu, Seoul, 139-956 (KR); HONG, Jung-Hwa, Chungcheongnam-do, 339-806 (KR); HWANG, Chang-Mo, Seongbuk-gu, Seoul,136-705 (KR); KIM, Byung-Jo, Seongbuk-gu Seoul 136-772 (KR)
(74) Representative: Rapisardi, Mariacristina
(86) International application number: PCT/KR2006/004683
(87) International publication number: WO 2007/055521

(56) References cited:
- EP-A- 0 521 595
- EP-B1- 0 754 437
- EP-B1- 0 959 786
- WO-A-02/089686
- FR-A- 2 694 700
- GB-A- 1 208 639
- KR-A- 20010 021 983
- US-A- 5 318 525
- US-A- 5 861 024
- US-A1- 2003 028 188
- US-A1- 2003 088 301
- US-A1- 2004 059 404
- US-A1- 2005 004 644
- US-B1- 6 416 509

## Description

### Technical Field

The present invention relates to a radiofrequency electrode for locally ablating a tissue in a specific region in a body using a radiofrequency (RF), and more specifically to a radiofrequency electrode which is used for easily ablating a tissue in a desired location, by inserting, through a catheter, a radiofrequency electrode tip assembly to a tissue region in a body to be ablated, and then controlling the direction of the electrode by an elastic force of an end part of the radiofrequency electrode.

### Background Art

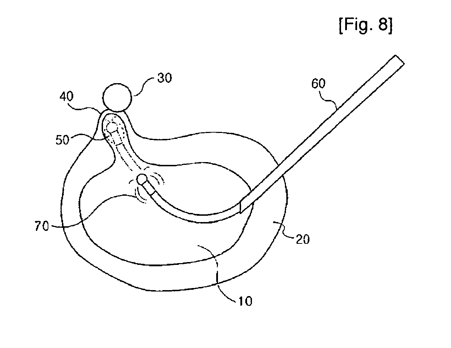

Generally, if a hernia of the intervertebral discs in the spine occurs, the intervertebral discs in the spine protrude to put pressure on an adjacent nerve, thus causing a lower back pain. The intervertebral discs are composed of a nucleus pulposus 10 (illustrated in Fig. 8) and an annulus fibrosus surrounding the nucleus pulposus 20 (illustrated in Fig. 8). If upon applying any physical impact to the intervertebral discs, the inner wall of the annulus fibrosus is torn, a pressure generated from the body weight or excessive impact generated when one stands up for a long period of time, causes the nucleus pulposus in the intervertebral discs to flow out between the torn inner walls of the annulus fibrosus, and a high pressure in the intervertebral discs is transferred to the skin of the intervertebral discs, whereby a part of the intervertebral discs is protruded. This phenomenon is referred to as a "hernia of the intervertebral discs." The protruded parts of the intervertebral discs do not restore their original states and continuously maintain their protruded forms, whereby pressure is put on the nerve near the spin, thereby leading to a lumbar pain.

The hernia of the intervertebral discs can be treated by surgical operation. However, a therapeutic effect cannot be attained in 30% of the surgically operated patients, and further the surgical operation is accompanied by ablation of the spinal nerve region, which causes 5 to 10% of the operated patients to suffer Failed Back Surgery Syndromes (F.B.S.S) for one's lifetime.

As an alternative approach for treating the hernia of the intervertebral discs, mention may be made of a restoring method comprising the steps of removing the components of the nucleus pulposus in the intervertebral discs to reduce the internal pressure of the intervertebral discs, which causes the protruded region of intervertebral discs to spontaneously get back to the inside of the intervertebral discs. This approach can be effected not only by a surgical operation, but also by a non-surgical operation, which is a method comprising the steps of inserting a radiofrequency electrode in a tissue in the intervertebral discs, and applying radiofrequency to ablate the tissues around the electrode by the high-energy magnetic field formed using radiofrequency in the gas state where the components of the nucleus pulposus are separated into the negatively charged electrons and the positively charged ions. The method for ablating a tissue by means of a radiofrequency electrode is advantageous in that it can make the hospital stays shorter, significantly lower a surgery cost, and lessen the risk of generation of post-surgery side-effects, as compared with the surgical operation method.

The radiofrequency is a frequency in the range from 100 to 20,000 kHz, and methods for ablating or removing a body tissue, and removing waste products, etc. in the blood vessel are disclosed in U.S. Patent No. 6,554,827 B2, etc.

Fig. 8 is a diagram illustrating the treatment of a hernia of the spinal intervertebral discs using a radiofrequency electrode. As illustrated in Fig. 8, a part of the intervertebral discs in a human body is protruded to put pressure on a nerve root 30, thus causing a waist pain. The skin 40 of the protruded part of the spinal intervertebral discs gets hydrostatic pressure by the internal composition 50 of the protruded part, and the internal composition 50 of the protruded part does not have high fluidity, and thus maintains its protruded form, while continuously putting pressure on the nerve root 30, thereby causing a lumbar pain. Here, by positioning the radiofrequency electrode tip through a catheter 60 in the internal composition 50 in the skin 40 of the protruded part of the spinal intervertebral discs, and then applying radiofrequency, a radiofrequency region is generated around two electrodes, and the tissues in the radiofrequency-applied part 70 around the electrodes are separated into the negatively charged electrons and the positively charged ions, and accordingly, are changed to ones in the neutral gas having a high degree of charge separation, and with a lowered pressure, the protruded part returns to the original state.

### Disclosure of Invention

### Technical Problem

Conventional radiofrequency electrodes are in the linear form, and thus electrodes are limited in the regions to be approached in a body, since the electrodes reach only the locations arranged in line with the locations in the body to which the electrode is inserted. Particularly, if the hernia of the intervertebral discs is treated, the presence of the spine and spinal nerve sets a limit on the locations to which a radiofrequency electrode tip is inserted. As such, as illustrated in Fig. 8, the tissue regions opposite to the locations to which the radiofrequency electrode is inserted is hard to be ablated. The method using a radiofrequency electrode adopts a principle of restoring the extruded region of the intervertebral discs by a negative pressure generated from ablation of the tissue, and thus it has extremely low efficiency in the case where the tissue around the extruded region cannot be ablated.

On the other hand, mention may be made of another method in which a radiofrequency electrode having its ends preliminarily curved at a prescribed curvature prior to be inserted from the outside is guided by a catheter, and then reaches to the parts, which is hard to be linearly approached, by means of a restoring force. In this method, however, the curves of the parts which had once entered a body are resided in a pre-determined fashion.

EP-A-0 521 595 discloses a radiofrequency electrode for selective ablation of a body tissue, comprising a first electrode and a second electrode which are adapted to be inserted into a body tissue, wherein the first electrode comprises a first electrode tip and a first electrode lead which is connected to the first electrode tip by its one end and the second electrode comprises a second electrode tip and a second electrode lead which is connected to the second electrode tip by its one end, and wherein the first electrode tip and second electrode tip are combined via an electrically insulating material to form an electrode tip assembly. Accordingly, the method has a risk of trials based on a experience or predictions, which gives a limit on use in the wide applications, and further it has a drawback that it is difficult to ablate the tissues using radiofrequency by precisely locating the electrode in the extruded region in the intervertebral discs which varies depending on the individuals.

Further, disclosed is a method in which an end of a radiofrequency electrode can be bent with a flexible polymeric material, but this method also has a drawback that it is difficult to precisely locate a radiofrequency electrode tip in the extruded region in the intervertebral discs, and the polymeric material is melted upon discharge of radiofrequency.

Therefore, the present invention has been made in order to overcome the above-described problems, and thus it is an object of the present invention to provide a radiofrequency electrode for selective ablation of a body tissue, which has a direction-controlling function so as to easily locate a radiofrequency electrode tip at a specific region in a body tissue.

### Technical Solution

The present invention relates to a radiofrequency electrode comprising a first electrode and a second electrode, each of which is guided to a body tissue by a catheter.

The radiofrequency electrode is a radiofrequency electrode for selective ablation of a body tissue, wherein a first electrode tip formed at the end of the first electrode and a second electrode tip formed at the end of the second electrode are combined with a predetermined gap between them. A coil spring is bonded to the ends of the first electrode and the second electrode, wherein the coil spring is configured to have its end part bent in the free state and is transformed by pulling either or both of the first electrode and the second electrode.

The first electrode comprises a first electrode tip and a first electrode lead, wherein the first electrode lead is connected to the first electrode tip, the first electrode lead is in the form of a metal wire, and inserted into the coil spring.

The second electrode comprises a second electrode tip and a second electrode lead, and the second electrode lead is in the form of a coil spring, wherein one end of the coil spring is connected to the second electrode tip.

The first electrode tip and the second electrode tip are combined with an electrically insulating material to form an electrode tip assembly.

One end of the second electrode tip is inserted into the electrically insulating material and fixed therein, and the other end is formed of a 'closely-wound coil spring' connected to the second electrode lead in the form of a coil spring.

The other end of the coil spring may be welded to one end of a metal tube, as guided by a catheter, to form a second electrode lead.

On the other hand, the first electrode comprises the first electrode tip and the first electrode lead, and the first electrode lead is in the form of a metal wire, wherein the first electrode lead is connected to the first electrode tip and is inserted into the coil spring. The second electrode comprises the second electrode tip and the second electrode lead, and the second electrode lead may be in the form of a metal wire, wherein the second electrode lead is connected to the second electrode tip, inserted into the coil spring, and disposed in parallel with the first electrode lead.

The first electrode tip and the second electrode tip are bonded by an electrically insulating material to form an electrode tip assembly, and one end of the coil spring may be fixed on the electrode tip assembly.

The part to which the coil spring is bonded is formed to be protruded in the electrode tip assembly, and one end of the coil spring may be fixed on the part to which the coil spring is bonded.

The other end of the coil spring may be fixed on one end of the tube.

The coil spring is made from metals, preferably metals such as stainless steel, common alloy steel, titanium steel wire, and shape memory alloy.

### Advantageous Effects

The radiofrequency electrode for selective ablation of a body tissue according to the present invention can be used for ablating a tissue in a body, by inserting, through a catheter, a radiofrequency electrode tip to a tissue, and then by controlling the direction and position of the electrode tip through pulling an electrode lead in the form a metal wire to modify the coil spring part, and thus easily positioning the electrode tip in a specific region.

### Brief Description of the Drawings

Fig. 1 is a perspective view of the radiofrequency electrode according to the first Example of the present invention.

Fig. 2 is a perspective view of separation of an external electrode and an internal electrode of Fig. 1.

Fig. 3 is a detailed view of the end part of the radiofrequency electrode of Fig. 1.

Fig. 4 is a perspective view illustrating an operator in a trigger type, to which the radiofrequency electrode of the present invention is bonded.

Fig. 5 is a state diagram illustrating the control of the direction and the position of the electrode tip using the radiofrequency electrode of the present invention.

Fig. 6 is a perspective view of the radiofrequency electrode according to the second Example of the present invention.

Figs. 7(a) to 7(c) are diagrams illustrating the assembly process of the radiofrequency electrode of Fig. 6.

Fig. 8 is a state diagram illustrating the treatment of the hernia of the intervertebral discs in the spine using radiofrequency.

<Brief Description on the Symbols in the Drawings>

100, 200: Radiofrequency electrode

110, 210: First electrode

112, 212: First electrode tip

114, 214: First electrode lead

120, 220: Second electrode

122, 222: Second electrode tip

124, 224: Second electrode lead

126: Metal tube

230: Coil spring

232: Metal tube

140, 240: Electrically insulating material

242: Bonded part of coil spring

150, 250: Electrode tip assembly

160: Operator

162: Trigger

60: Catheter

### Best Mode for Carrying Out the Invention

Hereinbelow, preferable Examples of the present invention will be described in detail with reference to the accompanying figures.

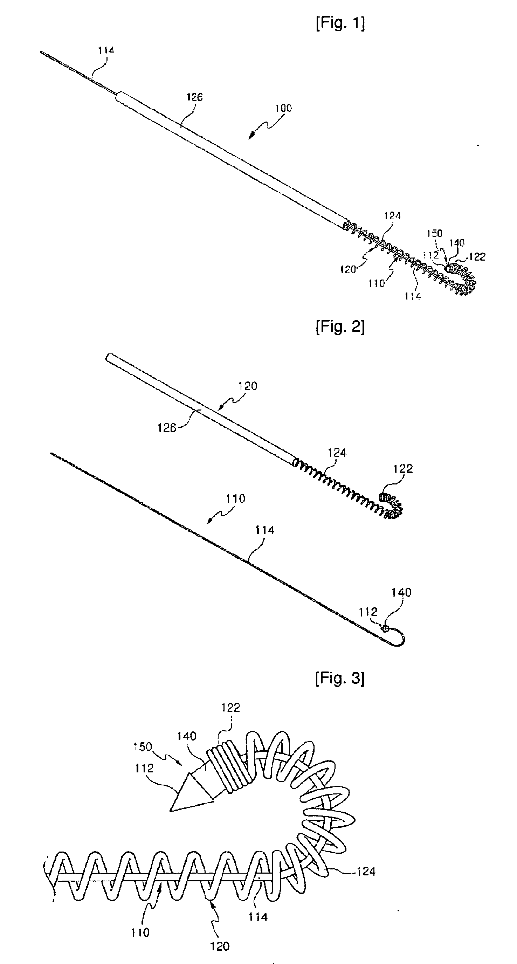

Fig. 1 is a perspective view of the radiofrequency electrode according to the first Example of the present invention, Fig. 2 is a perspective view of separation of an external electrode and an internal electrode of Fig. 1, and Fig. 3 is a detailed view of the end part of the radiofrequency electrode of Fig. 1. As illustrated in the figures, the radiofrequency electrode 100 of the first Example is configured such that the first electrode 110 in the form of a metal wire is inserted into the second electrode 120 having its end part in the form of a coil spring.

The first electrode 110 and the second electrode 120 are guided by a catheter 60 (illustrated in Fig. 8) and inserted into a body tissue.

The first electrode 110 is configured such that the first electrode lead 114 is inserted into the second electrode 120 and disposed therein, and the first electrode tip 112 in the sharp cylindrical form is connected to the end part of the first electrode lead 114. The first electrode lead 114 is coated by a well known insulator, and the first electrode tip 112 is not coated.

The second electrode 120 is configured such that the second electrode tip 122 in the form of a closely-wound coil spring is connected to the end part of the second electrode lead 124 in the form of a coil spring, and metal tube 126 is welded in the opposite side of the second electrode tip 122 to wholly form the second electrode 120. The second electrode lead 124 and the metal tube 126 are coated by a well known insulator, and the second electrode tip 122 in the form of a closely-wound coil spring is not coated. The second electrode 120 has an outer diameter (outer diameters of the metal tube and the coil spring) of 0.8 to 2.5 mm.

The first electrode tip 112 and the second electrode tip 122 are bonded to each other by a electrically insulating material 140 coated by an insulator in the cylindrical form to form an electrode tip assembly 150. The first electrode tip 112 is closely adhered and fixed on the side of the end part of the electrically insulating material 140, and the second electrode tip 122 is interposed and fixed on the other side of the electrically insulating material 140.

Further, in the second electrode lead 124 in the form of a coil spring, the end part connected to the second electrode tip 122 is bent in the free state, thus to be pigtail- or J-shaped.

The first electrode lead 114 is easily bent by an external force, and made from materials having a high tensile strength. When the first electrode lead 114 is inserted into the second electrode lead 124 in the form of a coil spring, the end part of the first electrode lead 114 is also bent to be pigtail- or J-shaped, according to the shape of the bent second electrode lead. The first electrode lead is connected to the trigger 162 of the below-described operator 160, and functions to control the direction of the electrode tip by the operation of the trigger 162.

Any materials for the first electrode, the second electrode, and the coil spring can be used, as long as radiofrequency current flows therein, preferably used are metals, and more preferably used are metals such as stainless steel, common steel alloy, titanium steel wire, and shape memory alloy.

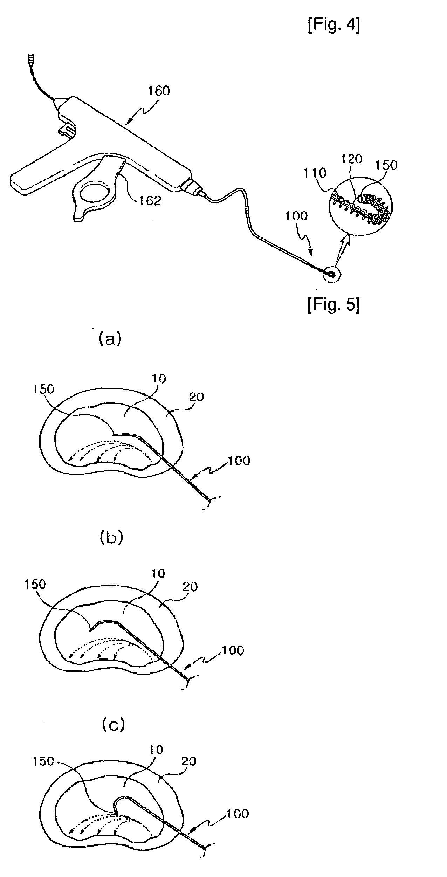

Such configured radiofrequency electrode 100 is, for example, connected to the operator 160 for use, as illustrated in Fig. 4. If the first electrode 110, which had been inserted into the second electrode 120 by operation of the trigger 162 in the operator 160, is pulled, the electrode tip assembly 150 is also pulled, the bent part (the pigtail-or J-shaped part) of the end part of the second electrode 120 in the form of a coil spring is unbent, and if the controller 162 is restored to its original position, the elastic force from the bent part of the second electrode 120 allows restoration of the bent form which is an original form, thus making it possible to control the position and the direction of the electrode tip assembly 150.

By such the operation, Figs. 5(a) to 5(c) illustrates a method in which an electrode tip assembly is inserted into the intervertebral discs to control the direction and the position of the electrode tip assembly.

As illustrated in Fig. 5, when the electrode tip assembly 150 is inserted into the annulus fibrosus 20 of the intervertebral discs, the controller 162 (illustrated in Fig. 4) is pulled to almost unfold the bent part of the end part of the radiofrequency electrode 100. After the electrode tip assembly 150 is inserted into the outer skin 20, if one slowly release the trigger 162 (illustrated in Fig. 4), the end part of the radiofrequency electrode 100 is again bent, as illustrated in Figs. 5(b) and 5(c). If the trigger 162 is pulled or released, and correspondingly the electrode tip is unbent or bent, the electrode tip assembly 150 can be easily positioned in the nucleus pulposus part 10 in the annulus fibrosus 20 in the direction and the position, as shown by the broken lined arrow, thus make it possible to easily perform the operation for applying radiofrequency.

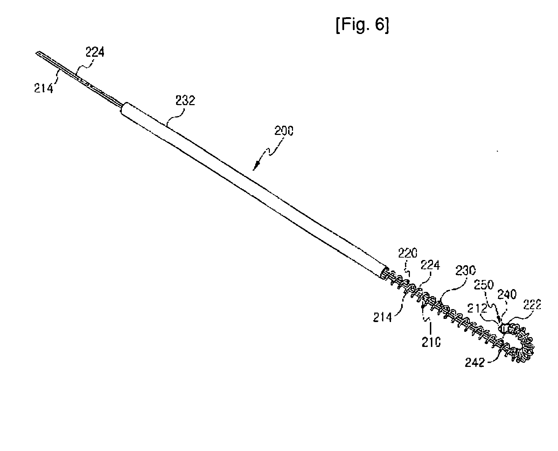

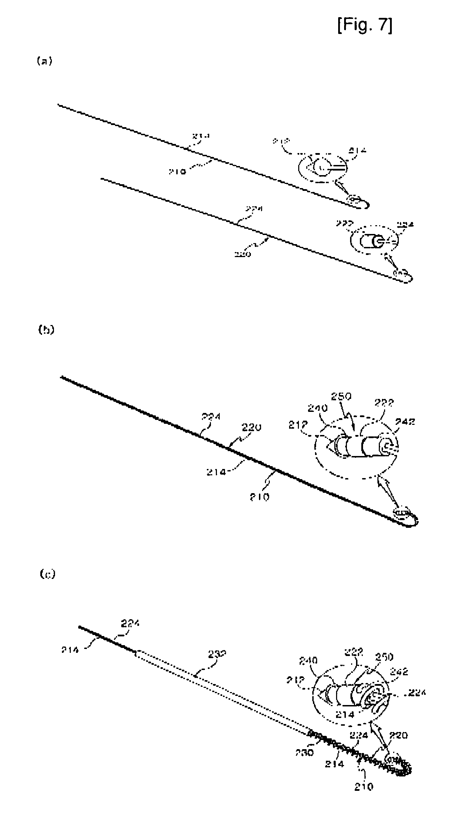

Fig. 6 is a perspective view of the radiofrequency electrode according to the second Example of the present invention, and Fig. 7 is a diagram illustrating the assembly process of the radiofrequency electrode of Fig. 6.

As illustrated in the figures, the radiofrequency electrode 200 of the second Example is configured such that a separate coil spring 230 is provided at the end part of the radiofrequency electrode, and the first electrode 210 in the form a metal wire and the second electrode 220 are inserted into the coil spring 230 and disposed in parallel to each other.

The first electrode 210 and the second electrode 220 are guided by the catheter 60 (illustrated in Fig. 8) and inserted into the body tissue. A coil spring 230, by which the first electrode 210 and the second electrode 220 are guided, is provided at the end parts of the first electrode 210 and the second electrode 220.

The first electrode 210 is configured such that the first electrode lead 214 in the form of a metal wire is inserted into the coil spring 230 and disposed therein, and the first electrode tip 212 in the sharp cylindrical form is connected to the end part of the first electrode lead 214. The first electrode lead 214 is coated by a well known insulator, and the first electrode tip 212 is not coated.

The second electrode 220 is configured such that the second electrode lead 224 in the form of a metal wire is inserted into the coil spring 230 and disposed in parallel with the electrode lead 214, and the second electrode tip 222 in the cylindrical form is connected to the end part of the second electrode lead 224. The second electrode lead 224 is coated by a well known insulator, and the second electrode tip 222 is not coated.

The first electrode tip 212 and the second electrode tip 222 are bonded to a electrically insulating material 240 in the cylindrical form coated by an insulator to form an electrode tip 250. The first electrode tip 212 is closely adhered to and fixed on the side of the end part of the electrically insulating material 240, and the second electrode tip 222 is interposed and fixed on the side of the other end part of the electrically insulating material 240. In the electrode tip 250, the cylindrical part 242 to which the coil spring is bonded, that is, the part to which the coil spring 230 is bonded, is protruded in the opposite side of the electrically insulating material 240, and the end part of the coil spring 230 is inserted into the outer surface of the part 242 to which the coil spring is bonded. The part 242 to which the coil spring is bonded is coated by a well known insulator.

In the coil spring 230, a metal tube 232 is welded to the opposite side of the electrode tip 250.

In the coil spring 230, the end part connected to the second electrode tip 250 is bent in the free state, thus to be pigtail- or J-shaped.

In the first electrode lead 214 and the second electrode lead 224, which are inserted into the coil spring 230, even the end part connected to the electrode tip 250 may be bent in the free state, thus to be pigtail- or J-shaped.

Any materials for the first electrode 210, the second electrode 220, the coil spring 230, and the tube 232 can be used, as long as radiofrequency current flows therein, preferably used are metals, and more preferably used are metals such as stainless steel, common steel alloy, titanium steel wire, and shape memory alloy. Thus prepared coil spring 230 and the tube 232 are coated by a well known insulator so as not to be affected upon applying radiofrequency between the first electrode tip 212 and the second electrode tip 222.

Figs. 7(a) to 7(c) illustrate the assembly process of the radiofrequency electrode of the second Example of the present invention. As illustrated in Fig. 7(a), the first electrode tip 212 and the first electrode lead 214 are assembled into a first electrode 210, while the second electrode tip 222 and the second electrode lead 224 are assembled into a second electrode 220. Thereafter, as illustrated in Fig. 7(b), an electrically insulating material 240 is bonded between the first electrode tip 212 and the second electrode tip 222, while the part 242 to which the coil spring is bonded is bonded in the opposite side of the electrically insulating material 240 of the second electrode tip 222 to form an electrode tip 250. Then, as illustrated in Fig. 7(c), the coil spring 230 having its end part bent is inserted and fixed to the part 242 to which the coil spring is bonded is bonded to obtain a radiofrequency electrode 200.

Thus configured radiofrequency electrode 200 according to the second Example of the present invention is bonded to a trigger-type operator 160 as illustrated in Fig. 4 for use, as in the radiofrequency electrode 100 according to the first Example of the present invention, and the operation using the operator is as illustrated in Figs. 5(a) to 5(c). That is, an electrode tip is inserted into the nucleus pulposus 10 of the intervertebral discs, and controlled in the direction and the position, wherein if the first electrode 210 and the second electrode 220, which had been inserted into the coil spring 230 by operation of the trigger 162 in the operator 160, are pulled, the electrode tip 250 is also pulled, the bent part (the J-shaped part of the end part of the coil spring 230 is unbent, and if the controller 162 is restored to its original position, the elastic force from the bent part of the coil spring 230 allows restoration of the bent form which is an original form, thus making it possible to control the position and the direction of the electrode tip 250.

In the second Example of the present invention, the first electrode lead 214 and the second electrode lead 224 are both in the linear forms, and the first electrode lead 214 and the second electrode lead 224 are configured to be located inside of a separate coil spring 230. Thus, the second electrode lead 124 is shorter than that in the coil form of the first Example, which allows a lower current loss due to resistance, and a separate coil spring 230 protects the first electrode lead 214 and the second electrode lead 224, which eliminates the noise due to outer currents or electromagnetic wave, thereby it provides an effect of shielding an electromagnetic wave.

According to the radiofrequency electrode of the present invention, local ablation can be performed with various applications, even in the case of the regions to which a linear electrode cannot be easily accessed, such as the hernia of the intervertebral discs. Further, if a local ablation of a body tissue in addition to the above-described cases is required, for example: to ablate a tumor or a cancer tissue; to ablate a thrombus in a blood vessel; to ablate a plaque in a blood vessel; to ablate a stenosed area in a blood vessel; to ablate a fibroma; to ablate a myoma of the uterus; to ablate an apocrine gland for alleviation of an osmidrosis; to ablate a polyp in the small or large intestine; to ablate a tissue in the stomach, small intestine or large intestine; to ablate a stenosed area in the urethra; to ablate a stenosed area in the knee cartilage; to ablate an undesirably grown nerve tissue; or the like.

On the other hand, the coil spring made from the metal materials in the present invention, as compared with the coil spring made from polymeric materials, can provide a spring back force with a predetermined curvature (pre-curve), and thus makes it possible to locally control the direction of the electrode tip, to perform a precise operation, to have heat resistance against a neutral gas separated into electrons and ions at around 700°C by radiofrequency, to eliminate the deterioration by the electrode in the case of controlling an electrode in the form of a metal wire, to perform insulation employing various coating techniques, and to have a low production cost.

## Claims

1. A radiofrequency electrode (100) for selective ablation of a body tissue, comprising a first electrode (110) and a second electrode (120) which arc adapted to be inserted into a body tissue, wherein the first electrode (110) comprises a first electrode tip (112) and a first electrode lead (114) which is connected to the first, electrode tip (112) by its one end and the second electrode (120) comprises a second electrode tip (122) and a second electrode lead (124) which is connected to the second electrode tip (122) by its one end, and wherein the first electrode tip (112) and second electrode tip (122) are combined via an electrically insulating material (140) to form an electrode tip assembly (150), **characterized in that** the second electrode lead (124) is in the form of a coil spring and the first electrode lead (114) is inserted into the coil spring, wherein a portion of the coil spring adjacent to the electrode tip (150) assembly is bent with respect to the remaining portion of the coil spring, and wherein the bent portion of the coil spring is configured to be at least partially unbent based on a tensile force applied to the first electrode (110).

2. The radiofrequency electrode (100) for selective ablation of a body tissue according to claim 1, wherein the second electrode tip (122) is in the form of a closely-wound coil spring, one end of the second electrode tip (122) being inserted into the electrically insulating material (140) and fixed therein, the other end of the second electrode tip (122) being connected to the second electrode lead (124) in the form of a coil spring.

3. The radiofrequency electrode (100) for selective ablation of a body tissue according to claim 1, wherein the other end of the second electrode lead (124) which is in the form of a coil spring is welded to one end of a metal tube (126).

4. The radiofrequency electrode (100, 200) for selective ablation of a body tissue according to any one of claims 1-3, wherein the coil spring is made from metal.

5. The radiofrequency electrode (100, 200) for selective ablation of a body tissue according to any one of claims 1-3, wherein the body tissue is at least one selected from the group consisting of a cancer tissue, a tumor tissue, a tissue in the blood vessel, a fibroma tissue, an apocrine gland, a stenosed tissue in the urethra, a stenosed tissue in the joint cartilage, and a tissue in the stomach, small intestine or large intestine.

## Patentansprüche

1. Hochfrequenzelektrode (100) zur selektiven Ablation von Körpergewebe, umfassend eine erste Elektrode (110) und eine zweite Elektrode (120), ausgelegt, um in ein Körpergewebe eingeführt zu werden, wobei die erste Elektrode (110) eine erste Elektrodenspitze (112) und einen ersten Elektrodendraht (114), der mit der ersten Elektrodenspitze (112) durch eines seiner Enden verbunden ist, umfasst, und die zweite Elektrode (120) eine zweite Elektrodenspitze (122) und einen zweiten Elektrodendraht (124), der mit der zweiten Elektrodenspitze (122) durch eines seiner Enden verbunden ist, umfasst, und wobei die erste Elektrodenspitze (112) und die zweite Elektrodenspitze (122) über ein elektrisch isolierendes Material (140) kombiniert sind, um eine Elektrodenspitzengruppe (150) zu bilden, **dadurch gekennzeichnet, dass** der zweite Elektrodendraht (124) die Form einer Spiralfeder aufweist und der erste Elektrodendraht (114) in die Spiralfeder eingesetzt ist, wobei ein Abschnitt der Spiralfeder, angrenzend an die Elektrodenspitzengruppe (150), im Vergleich zum restlichen Abschnitt der Spiralfeder gebogen ist, und wobei der gebogene Abschnitt der Spiralfeder so ausgestaltet ist, dass er mindestens teilweise nicht gebogen ist, basierend auf der auf die erste Elektrode (110) angewandten Zugkraft.

2. Hochfrequenzelektrode (100) zur selektiven Ablation von Körpergewebe nach Anspruch 1, wobei die zweite Elektrodenspitze (122) die Form einer eng gewundenen Spiralfeder aufweist und ein Ende der zweiten Elektrodenspitze (122) in das elektrisch isolierendes Material (140) eingesetzt und darin fixiert ist und das andere Ende der zweiten Elektrodenspitze (122) mit dem zweiten Elektrodendraht (124) in der Form einer Spiralfeder verbunden ist.

3. Hochfrequenzelektrode (100) zur selektiven Ablation von Körpergewebe nach Anspruch 1, wobei das andere Ende des zweiten Elektrodendrahts (124), der die Form einer Spiralfeder aufweist, an einem Ende eines Metallrohrs (126) angeschweißt ist.

4. Hochfrequenzelektrode (100, 200) zur selektiven Ablation von Körpergewebe nach einem der Ansprüche 1 bis 3, wobei die Spiralfeder aus Metall besteht.

5. Hochfrequenzelektrode (100, 200) zur selektiven Ablation von Körpergewebe nach einem der Ansprüche 1 bis 3, wobei es sich beim Körpergewebe mindestens um eins handelt, das aus der Gruppe, bestehend aus einem Krebsgewebe, einem Tumorgewebe, einem Gewebe im Blutgefäß, einem Fibromgewebe, einer apokrinen Drüse, einem Stenose-Gewebe in der Harnröhre, einem Stenose-Gewebe im Gelenkknorpel und einem Gewebe im Magen, dem Dünndarm oder dem Dickdarm, ausgewählt ist.

## Revendications

1. Electrode à fréquence radio (100) pour l'ablation sélective d'un tissu cellulaire, comprenant une première électrode (110) et une seconde électrode (120) pouvant être insérées dans un tissu cellulaire, dans laquelle la première électrode (110) comprend une première pointe d'électrode (112) et une première tige d'électrode (114) qui est reliée à la première pointe d'électrode (112) par une de ses extrémités et la seconde électrode (120) comprend une seconde pointe d'électrode (122) et une seconde tige d'électrode (124) qui est reliée à la seconde pointe d'électrode (122) par une de ses extrémités, et dans laquelle la première pointe d'électrode (112) et la seconde pointe d'électrode (122) sont combinées via un matériau électriquement isolant (140) pour former un assemblage de pointes d'électrode (150), **caractérisée en ce que** la seconde tige d'électrode (124) a la forme d'un ressort hélicoïdal et la première tige d'électrode (114) est insérée dans le ressort hélicoïdal, dans laquelle une partie du ressort hélicoïdal adjacente à l'assemblage de pointes d'électrode (150) est pliée par rapport à la partie restante du ressort hélicoïdal, et dans laquelle la partie pliée du ressort hélicoïdal est configurée pour être au moins partiellement redressée par l'action d'une force de traction appliquée sur la première électrode (110).

2. Electrode à fréquence radio (100) pour l'ablation sélective d'un tissu cellulaire selon la revendication 1, dans laquelle la seconde pointe d'électrode (122) a la forme d'un ressort hélicoïdal enroulé de façon serrée, une extrémité de la seconde pointe d'électrode (122) étant insérée dans le matériau électriquement isolant (140) et fixée sur celui-ci, l'autre extrémité de la seconde pointe d'électrode (122) étant reliée à la seconde tige d'électrode (124) en forme de ressort hélicoïdal.

3. Electrode à fréquence radio (100) pour l'ablation sélective d'un tissu cellulaire selon la revendication 1, dans laquelle l'autre extrémité de la seconde tige d'électrode (124) étant en forme de ressort hélicoïdal est soudée à une extrémité d'un tube de métal (126).

4. Electrode à fréquence radio (100, 200) pour l'ablation sélective d'un tissu cellulaire selon l'une quelconque des revendications de 1 à 3, dans laquelle le ressort hélicoïdal est en métal.

5. Electrode à fréquence radio (100, 200) pour l'ablation sélective d'un tissu cellulaire selon l'une quelconque des revendications de 1 à 3, dans laquelle le tissu cellulaire est au moins l'un de ceux compris dans le groupe suivant : un tissu cancéreux, un tissu tumoral, un tissu de vaisseau sanguin, un tissu de fibrome, un tissu de glande apocrine, un tissu sténosé dans l'urètre, un tissu sténosé dans le cartilage articulaire et un tissu pouvant se trouver dans l'estomac, l'intestin grêle ou le gros intestin.
